# EUROPEAN PATENT SPECIFICATION

(11) **EP 1 980 244 B1**
(45) Date of publication and mention of the grant of the patent: **07.08.2013**
(21) Application number: 08007212.7
(22) Date of filing: 11.04.2008
(51) Int. Cl.: A61K 9/14, A61K 9/26

(54) **Basis particles and orally-disintegrating tablet containing them**
Basispartikel und im Mund zerfallende Tablette, die dieselben enthält
Particules de base et comprimé se délitant en bouche les contenant

(30) Priority: 12.04.2007 JP 2007104477
(43) Date of publication of application: 15.10.2008
(73) Proprietor: NIPRO CORPORATION, Osaka-shi, Osaka 531-8510 (JP)
(72) Inventor: Sakuragi, Shiho, Kusatsu-shi Shiga-ken 525-0055 (JP); Hoashi, Yohei, Kusatsu-shi, Shiga-ken 525-0055 (JP); Katayama, Naohisa, Kusatsu-shi Shiga-ken 525-0055 (JP); Kai, Toshiya, Kusatsu-shi Shiga-ken 525-0055 (JP); Ohmichi, Noami, Kusatsu-shi Shiga-ken 525-0055 (JP)
(74) Representative: Schüssler, Andrea

(56) References cited:
- WO-A1-00/01369
- WO-A1-98/50019
- WO-A1-2004/075881
- WO-A1-2007/102714
- WO-A2-01/52848
- WO-A2-2006/105798
- WO-A2-2007/017219
- WO-A2-2007/090091
- DE-A1-102005 032 806

## Description

### 1. Field of the Invention

The present invention relates to basis particles, a method for manufacturing the same, and orally-disintegrating tablets, and more specifically relates to basis particles capable of masking bitterness, a method for manufacturing the same, and orally-disintegrating tablets.

### 2. Description of Related Art

The dosage form most generally adopted in oral solid preparations is the tablet form. Conventional pharmaceutical preparations disintegrate in the digestive tract such as stomach, if they are swallowed soon after ingestion since they disintegrate after two minutes or more.
However, orally-disintegrating tablets, which are easily swallowed by the young and elderly, that is, emphasize increasing patient quality of life (QOL), are noted as a dosage form that may cause an unpleasant sensation due to the bitterness of a basis (i.e., a main ingredient or an active drug) at the time of ingestion as they disintegrate intraorally within 30 seconds of ingestion, although they can be ingested without water.

The development of technology that makes it easier for a basis to be absorbed by the stomach or intestines and technology for masking the bitter taste of the basis has therefore been taking place. As the simplest of these methods, technology to conceal the bitterness by the addition of sweetners such as aspartame, stevia, or sugar alchohol and the addition of a flavoring such as L-menthol is proposed (see, for example, Japanese Laid-Open Patent Application H8-208517, H10-101582, 2001-302510 and 2001-106639).

However, since this technology conceals the bitterness with other flavors, complete elimination of drug bitterness is difficult.
Technology where the basis is coated with a coating agent has therefore been proposed (see, for example, Japanese Laid-Open Patent Application 2005-60309).
With this technology, it is disclosed that bitterness is suppressed by directly coating the basis with a water-insoluble coating film.

However, with this technology, delays in elution are observed depending on the drug, and a phenomenon may occur where the amount of drug originally included is not eluted. When this tendency is observed in elution tests, elution occurs without problems in the case of a basic drug in an eluate at an acidic pH but delays in elution occur with an eluate of a pH of 5.0 or more.

When coated pharmaceutical preparations are ingested in cases where the pH within the stomach is high as a result of achlorhydria or diet, elution of the drug is insufficient and satisfactory results cannot be obtained.

### SUMMARY OF THE INVENTION

It is an object of the present invention to provide a basis (i.e., a main ingredient or an active drug) particles and method for manufacturing the same as well as an orally-disintegrating tablet with which superior elution can be exhibited without dependence on bodily pH and despite characteristics of the acidic and basic basis, and the bitterness of the basis can be masked.

The present invention provides a basis particle comprising a basic or acidic basis particle coated by a water-insoluble coating film, wherein the water-insoluble coating film contains a substance that is acidic with respect to the basic basis or basic with respect to the acidic basis.
Further, the present invention provides a method for manufacturing a basis particle comprising: coating a basic or acidic basis particle with a water-soluble primary coating film containing a substance that is acidic with respect to the basic basis or basic with respect to the acidic basis, and coating the basis particle obtained with a water-insoluble coating film.
Moreover, the present invention provides an orally-disintegrating tablet comprising the basis particle of the above, wherein the tablet is tabletted with a pharmaceutically acceptable excipient, disintegrant and/or lubricant.

According to the basis particles and method for manufacturing the basis (i.e., a main ingredient or an active drug) particles of the present invention, it is possible to temporarily adjust pH occurring in the immediate proximity of the basis particles by using a coating film, elution of the basis particles is suppressed and superior elution is exhibited without dependence on bodily pH. It is also possible to mask tastes such as the bitterness of the basis and it is possible to ingest drugs without sensing any bitterness.

According to orally-disintegrating tablets of the present invention, it is possible to provide pharmaceutical preparations without fear of affecting the therapeutic effects even in achlorhydric patients or when the drug is ingested postprandially.

### BRIEF DESCRIPTION OF THE DRAWINGS

FIGs. 1A and 1B are graphs showing a drug elution behavior of the basis particles and the orally-disintegrating tablet of the present invention, respectively;
FIGs. 2A and 2B are graphs showing a drug elution behavior of the basis particles and the orally-disintegrating tablet of the comparative example, respectively.

### DETAILED DESCRIPTION OF THE PREFERRED EMBODIMENTS

The present invention is constructed to comprise a basis particle and a water-insoluble coating film coating at least the basis particle.
There is no particular restriction on the basis particles used in the present invention, but it can be the basis particles themselves having basic or acidic property. Examples include medicines for airsickness, analgesic antipyretic, aromatic stomachic, digestant, antacid agent, vitamin, nutrient tonic, enzyme preparation, nutrient tonic supplement, anti-inflammatory, antirheumatic drug, gout remedy, antihistamine, allergy agent, antibiotic agent, synthetic antibacterial, medicine for dental and oral use, bronchodilator, cough remedy, expectorant drug, sleep sedative, anxiolytic agent, antiepileptic drug, psychoneurotic agent, autonomic agent, central nervous system drug, antispasmodic agent, ameliorant of cerebral metabolism, ameliorant of cerebral circulation, antiParkinson's disease agent, Alzheimer therapeutic agent, cardiotonic agent, antiarrhythmia agent, diuretic agent, vasoconstrictor, vasodilatation agent, hypotensive agent, antihyperlipemia agent, constipating agent, peptic ulcer agent, cathartic, hormone agent and diabetes agent.

In particular, examples of the basic basis include substances having, for example, an amino group (primary, secondary, tertiary, or quaternary), nifedipine, nitrendipine, amlodipine besilate, risperidone, zolpidem tartrate, donepezil hydrochloride, diclofenac sodium, loxoprofen sodium and ibuprofen. Examples of the acidic basis include acetaminophen and ascorbic acid. These can also be in the form of a salt such as a free base, a hydrochloride, and a sulfate.

From a different viewpoint, the basis can be drug having bitterness. Example of the basis having bitterness include nifedipine, nitrendipine, amlodipine besilate, risperidone, zolpidem tartrate, donepezil hydrochloride, diclofenac sodium, loxoprofen sodium, ibuprofen and acetaminophen. The basis particles of the present invention are therefore particularly useful for the basic basis because a bitter drug tends to have basicity.

The basis can be powdered, solid, granular, and the like. There are no particular restrictions with regards to size, and the size can, for example, be varied as appropriate taking texture etc. into consideration when ingesting as an orally-disintegrating tablet. Specifically, a mean particle diameter of about 5 µm to about 50 µm may be shown as an example. A method of arranging particle diameters using, for example, a sieve or a membrane filter etc., or a method of crushing using a ball mill pulverizer, hammer mill pulverizer, or pin mill pulverizer can be given as methods for making the basis an appropriate shape and size. The basis can also be granulated using a known method in the field.

As the water-insoluble coating film for coating the basis particles, any of the various water-insoluble coating film which is commonly used in the field can be used. Here, as defined by the Japanese Pharmacopoeia, "water-insoluble" means extremely difficult to dissolve (the quantity of solvent required to dissolve 1g of solute is from 1000 ml or more to less than 10000 ml) and hardly dissolves at all (10000 ml or more). There is no particular restriction on the coating agent for forming the water-insoluble coating film, it can be used, for example, ethylcellulose, methacrylic acid co-polymer, amino methacrylic acid methacrylate co-polymer and hydroxypropylmethylcellulose phthalate. These can be used alone or as mixture of two or more.

There is no particular restriction on the film thickness of the water-insoluble coating film, for example, about 0.01 µm to about 20 µm. Further, from a further point of view, the water-insoluble coating film is preferably formed so as to be an extent of about 15 % to about 80% by weight with respect to the whole weight of the basis particles. It is therefore appropriate for a mean particle diameter of the basis particles of the present invention coated by the water-insoluble coating film to be varied between, for example, about 50 µm to about 300 µm, and preferably about 50 µm to about 200 µm.
An acidic substance with respect to the basic basis, or a basic substance with respect to an acidic basis is included at an inner part of the water-insoluble coating film, in other words, on the inside of the coating film. This means that the basis and the basic or acidic substance are present together at the space coated by the water-insoluble coating film.

It is therefore preferable that a substance that does not affect the effectiveness of the basis is adopted as the basic or acidic substance such as, for example, a substance used as a pH adjuster. Examples of the acidic substance include, for example, an organic acid such as citric acid, fumaric acid, succinic acid, acetic acid, tartaric acid, or salts thereof, and an inorganic acid such as hydrochloric acid, sulfuric acid, hydrobromic acid, phosphoric acid or salts thereof. Among these, it is preferably citric acid, acetic acid, tartaric acid, or salts thereof as the acidic substance. Examples of the basic substance include, for example, sodium hydroxide, sodium carbonate, sodium hydrogencarbonate and ammonia. These can be used alone or as mixture of two or more.

The quantity of the basic or acidic substance contained in the water-insoluble coating film can be varied appropriately according to the kind of basis, the kind of the basic or acidic substance and, for example, about 0.1 % to about 20 % by weight with respect to the total weight of the basis particles is appropriate. By adjusting this range, at the space enclosed within the water-insoluble coating film, it is possible to adjust the pH of the basis appropriately using slight permeation of fluid in the initial stage. The basis is therefore eluted in an appropriate manner under an environment such as in the stomach and the intestines.

The specific form of the basic or acidic substance being contained within (inside) the water-insoluble coating film may refer to existing between the basis particles and the water-insoluble coating film, being mixed with the basis particles, or the like.
More specifically, an example form is shown where, within the water-insoluble coating film, the basis particles are coated by a water-soluble primary coating film, and the water-soluble primary coating film includes the acidic substance with respect to the basic basis, or basic substance with respect to the acidic basis.

As the water-soluble primary coating film, any of the various water-soluble coating film which is commonly used in the field can be used. Here, as defined by the Japanese Pharmacopoeia, "water-soluble" means moderately-soluble (the quantity of solvent required to dissolve 1g of solute is from 10 ml or more to less than 30 ml), easily-soluble (from 1 ml or more to less than 10 ml) and extremely easily-soluble (less than 1 ml). The water- soluble primary coating film may include a film which is caused to swell by water and also results in a clear or slightly cloudy fluid having consistency as with hydroxypropyl methylcellulose described later. There is no particular restriction on a coating agent for forming the water-soluble primary coating film, but examples include hydroxypropylmethylcellulose, methylcellulose and hydroxypropyl cellulose. These can be used alone or as mixture of two or more.

There is no particular restriction on the film thickness of the water-soluble primary coating film, but it is suitably about 0.01 µm to about 20 µm, for example. Further, from a further point of view, the water-soluble primary coating film is preferably formed so as to an extent of about 3 % to about 15% by weight with respect to the whole weight of the basis particles.
The amount of the basic or acidic substance contained in the water-soluble primary coating film can be, for example, provided of 1 % to 30 % by weight (not including water) with respect to the total weight of the water-soluble primary coating film.
In a further form, when a mixture of the basis particles and the acidic or basic substance exists within the water-insoluble coating film, as described above, the granulated or ungranulated acidic or basic substance can exist together with the granulated or ungranulated basis particles, or the basis and acidic or basic substance can be mixed together and granulated to form a granules.

The granules can be formed using a known method in the field such as, for example, wet granulation and dry granulation, optionally, together with an additive for granulation. For example, granulation can be performed using various apparatus for wet granulation such as a fluidized bed granulation dehydrator, an aggregate granulating machine, a cylindrical extrusion aggregating machine, and a roll fluidized bed granulated coating machine, or various apparatus for dry granulation using spray dry techniques such as dry granulation machines, e.g., roller compactors, and slag tablet machines.

In a method for manufacturing a basis particles of the present invention, first, a basis particles are coated with a water-soluble primary coating film containing an acidic substance with respect to a basic basis, or a basic substance with respect to an acidic basis.
Any known method in the field can be utilized as the method for coating the basis particles with the water-soluble primary coating film. Particularly, a coating agent constituting the water-soluble primary coating film may be dissolved in a solvent such as water, and then, the basis particles may be coated with the water-soluble primary coating film using by a known method such as pan coating, flow coating, and rolling coating utilizing apparatus such as an inclined type pan, an aeration type rotating cylinder coating apparatus, a general purpose flow coating apparatus, a Worcester-type coating apparatus, and a composite rolling/flow coating apparatus. Also, it may be utilized a pump, a squirt gun, a solution sending line, and the like.

The coating agent may optionally includes a known additives in the field, such as a plasticizer, a sweetener, a dispersion stabilizer, an excipient and a lubricant.
Examples of the plasticizer include triethyl citrate, triacetin, ziacetin, acetylated monoglyceride, dibutyl sebacate, medium-chain triglyceride and dibutyl adipate.
Examples of the dispersion stabilizer include sodium polyphosphate and trisodium citrate.
As the sweetener, excipient or lubricant can be used the same agents as described bellow.

Next, the particles coated with the water-soluble primary coating film are coated with the water-insoluble coating film.
Any known method in the field can be utilized as the method for coating the basis particles obtained with the water-insoluble coating film. For example, the same method as the covering method described above other than using an organic solvent can also be given.

Basis particles obtained in this way can then be made into a pharmaceutical preparation in various ways. For example, other forms such as tablet, orally-disintegrating tablet, capsule, pill, troche, granule, powder, suspension, emulsion, liquid, syrup, ant the like provided orally are also possible. Among these, tablet form, particularly orally-disintegrating tablet, is useful.

These pharmaceutical preparations, particularly orally-disintegrating tablets, can be made by tableting together with commonly used additives.
Examples of the additive include excipient, disintegrating agent, lubricant, binder, solubilizing agent, fluidiser, sweetener, fragrance, foaming agent, surfactant, preservative and coloring agent.
Examples of the excipient include glucose, fructose, lactose, sucrose, hydrogenated maltose and sugar alcohol (for example, D-mannitol, erythritol, sorbitol, xylitol, trehalose, maltitol, lactitol, etc.). These can be used alone or as mixture of two or more.

Examples of the disintegrating agent include crospovidone, sodium starch glycolate, sodium carboxymethyl starch, starch, partially pregelatinized starch, cornstarch, lactose, calcium carbonate, precipitated calcium carbonate, calcium citrate, light silicic anhydride, synthetic aluminum silicate crystalline cellulose, low substitution degree hydroxypropylcellulose, croscarmellose, sodium croscarmellose, calcium carboxymethylcellulose, carmellose and hydroxypropyl starch. These can be used alone or as mixture of two or more. Among these, it is preperably croscarmellose, sodium starch glycolate, carmellose, starch, hydroxypropyl starch and crospovidone. The amount of the disintegrating agent can be, for example, provided of about 0.1 % or more, preferably about 0.5 % or more, and more preferably about 2 % or more by weight with respect to the total weight of the orally-disintegrating tablet. Also, it can be included about 30 % or less, preferably about 25 % or less, and more preferably about 15 % by weight.

Examples of the lubricant include magnesium stearate, calcium stearate, talc, sucrose fatty acid ester, polyethyleneglycol, stearic acid, light silicic anhydride, hydrogenated rape oil, hydrogenated castor oil, glycerin fatty acid ester, sodium stearyl fumarate, sodium benzoate, L-leucin and L-valine. These can be used alone or as mixture of two or more.

Examples of the binder include a water-soluble substance such as gelatine, agar, alginic acid, sodium alginate, dextrin, xanthan gum, arabian gum, hydroxyethylcellulose, hydroxypropylcellulose, hydroxypropylmethylcellulose, polyvinylpyrrolidone, partially saponification polyvinyl alcohol, methylcellulose, pullulan, partially pregelatinized starch and sugar. These can be used alone or as mixture of two or more.

Examples of the solubilizing agent include magnesium oxide, calcium oxide, sodium citrate, magnesium chloride, sodium carbonate and sodium bicarbonate. These can be used alone or as mixture of two or more.
Examples of the fluidizer include hydrated silicon dioxide and light silicic anhydride.
Examples of the sweetener include aspartame, sodium saccharin, dipotassium glycyrrhizinate, stevia and thaumatin. These can be used alone or as mixture of two or more.

Examples of the fragrance include mint, lemon or orange extract.
Examples of the foaming agent include tartrate, citrate and bicarbonate.
Examples of the surfactant include an anionic surfactant such as sodium alkylsulfate; a nonionic surfactant such as sucrose fatty acid ester, polyoxyethylene sorbitan fatty acid ester, polyoxyethylene fatty acid ester and polyoxyethylene castor oil derivatives. These can be used alone or as mixture of two or more.

Examples of the preservative include benzoic acid, parahydroxybenzoic acid, or a salt thereof.
Examples of the coloring agent include yellow oxide of iron, yellow iron sesquioxide, iron sesquioxide (red), orange essence, brown iron oxide, caramel, light silicic anhydride, Food Blue No.5, Food Yellow No.4, Food Yellow No.4 aluminum lake, Food Yellow No.5, Food Red No.2 (Amaranth), Food Red No.3, Food Red No.102, talc, sodium tetrafluorescein, green tea powder and Vitamin C.

The tableting can be carried out using a known apparatus employing a known method in the field. For example, a hydraulic hand press machine, a single punch tableting machine, a rotary type tableting machine, which are provided with a tableting mill, upper and lower pestles for tabletingcan be utilized as apparatus for tableting and compression molding.

Tableting requires regulation so that the tablets obtained have the appropriate hardness and rapidly disintegrate as orally-disintegrating tablets. There is no particular restriction on the tablet compression pressure, but it can be adjusted appropriately according to the apparatus used, the theory, the size of the tablets, and the type of basis, etc. In the case of using the apparatus described above, for example, a tablet compression pressure is suitably about 50kg/cm² or more, and about 1500 kg/cm² or less, preferably about 300 kg/cm² or more, and 1000 kg/cm² or less.

There is no particular restriction on the shape of the orally-disintegrating tablet of the present invention, but it includes a disc-shaped, a donut shaped, a polygonal plate-shaped, a spherical, an ellipsoidal, a caplet-shaped, and the like. It is preferable for the shape of the tablets to be the common disc-shape. There is no particular restriction on the size, but it is preferably a slightly larger size to the extent that swallowing does not take place directly, for example, a diameter of about 3 mm to about 30mm, and a thickness of about 1 mm to about 10mm.

The following is a description of an example of basis particles, a method for manufacturing basis particles, and orally-disintegrating tablets of the present invention.

### Example 1

5.3g of hydroxypropyl methylcellulose, 0.96g of citric acid anhydride, and 0.53g of polyethelene glycol (PEG-6000) was dissolved in 73.21g of purified water to give a coating fluid 1.
9.12g of triacetin was added to 121.5g of 30 % ethylcellulose dispersion fluid (CX-1), the obtained mixture was stirred for dispersion by agitation. And then, 2.44g of D-mannitol and 106.94g of purified water was added to the mixture, and stirred for dispersion to give a coating fluid II.

300g of blended powder of zolpidem tartrate: D-mannitol at a ratio of 1:1 was introduced into Worcester-type fluid bed granulating machine (MP-SPC-01, Burridge Engineering), and coating was performed firstly under the conditions of an intake air temperature of 75 degrees centigrade, an intake air flow of 0.40 m³/sec, an atomized air flow of 15.0 to 22.5 NL/minute, and a spray velocity for the coating fluid I of 4.5 to 7.8 g/minute.
And then, another coating was performed under the conditions of an intake air temperature of 75 degrees centigrade, an intake air flow of 0.35 m³/sec, an atomized air flow of 17.5 NL/minute, and a spray velocity for the coating fluid II of 6.0 to 8.0 g/minute to give basis particles I having a mean particle diameter of 150 µm.

24 g of the basis particles I, 20 g of microcrystalline cellulose, 5 g of anhydrous calcium hydrogen phosphate, 25 g of D-mannitol, 14 g of mannitol for direct impacting (PARTECH 100M, Merck Pharmaceuticals, granulated mannitol, mean granule diameter 90 to 120 µm), 10 g of crospovidone, 1 g of aspartame, and 1 g of magnesium stearate warer mixed together. Tableting was then carried out at a rotary tableting machine to give 100 mg tablets of a hardness of 40 N constituting orally-disintegrating tablets that disintegrate in twenty seconds within the mouth.

### Comparative Example 1

11.43 g of triacetin was added to 152.4 g of 30 % ethylcellulose dispersion fluid (CX-1), the obtained mixture was stirred for dispersion by agitation. And then, 2.85 g of D-mannitol and 133.32 g of purified water was added to the mixture, and stirred for dispersion to give a coating fluid.
300g of blended powder of zolpidem tartrate: D-mannitol at a ratio of 1:1 was introduced into Worcester-type fluid bed granulating machine (MP-SPC-01, Burridge Engineering), and coating was performed under the conditions of an intake air temperature of 75 degrees centigrade, an intake air flow of 0.35 m³/sec, an atomized air flow of 17.5 NL/minute, and a spray velocity for the coating fluid of 6.0 to 8.0 g/minute to give basis particles II having a mean particle diameter of 112 µm.

### Test Example 1

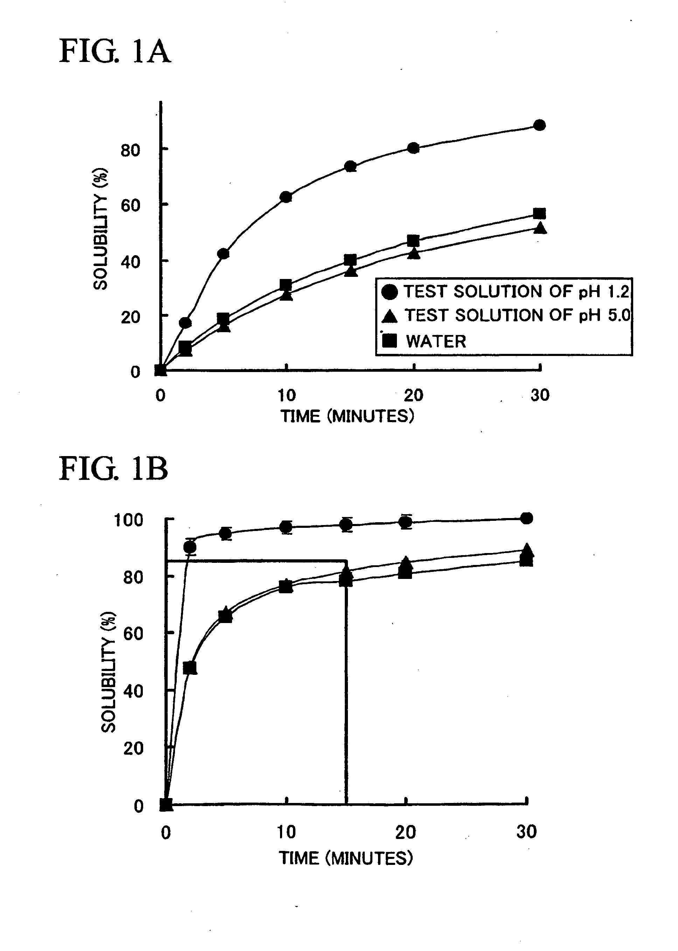

Elution test according to the rules for elution test of the Japanese Pharmacopoeia were then carried out for the basis particles I obtained in the Example 1 and the orally-disintegrating tablet made using the basis particles I. The test solutions were of pH1.2, pH5.0, and water. The results are shown in FIG. 1A and FIG. 1B. In FIG. 1A and FIG. 1B, the black circles denote a test solution of pH 1.2, the black triangles denote a test solution of pH 5.0, and the black squares denote water.
According to FIG. 1, it is observed that the basis particles of the present invention elute sufficiently in test solutions whatever the pH, and this does not depend on the pH. Further, rapid elution is confirmed regardless of the pH for tablet that disintegrate within the mouth. Bitterness is also suppressed sufficiently when the orally-disintegrating tablets disintegrate within the mouth.

### Test Example 2

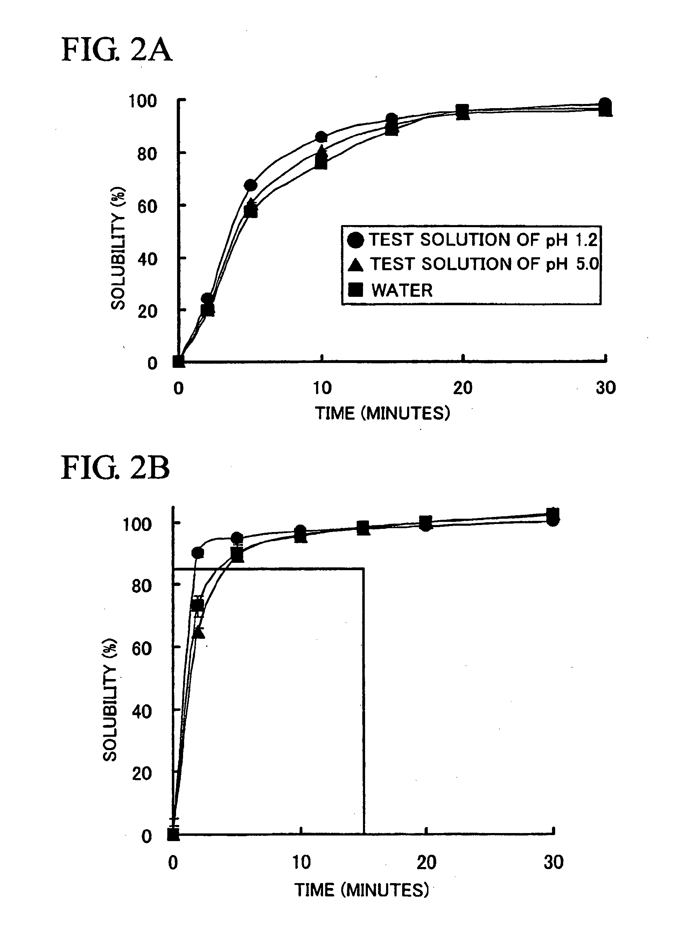

Elution test according to the rules for elution test of the Japanese Pharmacopoeia were then carried out for the basis particles II obtained in the Comparative Example 1 and the orally-disintegrating table made using the basis particles II. The test solutions are of pH1.2, pH5.0, and water. The results are shown in FIG. 2A and FIG. 2B.
According to FIG. 2, it is observed that there is a striking delay in elution for the basis particles II when the pH is high at pH 5.0 and in water. Similarly, a striking delay in elution can also be observed for a high pH of 5.0 and for water for the orally-disintegrating table.

The present invention can be utilized in various pharmaceutical drug preparations in order to conceal any taste or flavor, not just bitterness. Further, it is also possible to use any pharmaceutical drug preparation with the intent of adjusting solubility with respect to a basic or acidic drug.

## Claims

1. A basis particle comprising a basic basis particle coated by a water-soluble primary coating film and a water-insoluble coating film in this order,
wherein the primary coating film is formed by or contains at least one compound selected from the group consisting of citric acid, acetic acid, tartaric acid and a salt thereof and at least one compound selected from the group consisting of hydroxypropylmethylcellulose, methylcellulose and hydroxypropyl cellulose, and the water-insoluble coating film is formed by or contains at least one compound selected from the group consisting of ethylcellulose, methacrylic acid co-polymer, amino methacrylic acid methacrylate co-polymer and hydroxypropylmethylcellulose phthalate.

2. A method for manufacturing a basis particle comprising:
coating a basic basis particle with a water-soluble primary coating film containing at least one compound selected from the group consisting of citric acid, acetic acid, tartaric acid and a salt thereof and at least one compound selected from the group consisting of hydroxypropylmethylcellulose, methylcellulose and hydroxypropyl cellulose and coating the basis particle obtained with a water-insoluble coating film containing at least one compound selected from the group consisting of ethylcellulose, methacrylic acid co-polymer, amino methacrylic acid methacrylate co-polymer and hydroxypropylmethylcellulose phthalate.

3. An orally-disintegrating tablet comprising the basis particle of Claim 1, wherein the tablet is tabletted with a pharmaceutically acceptable excipient, disintegrant and/or lubricant.

## Patentansprüche

1. Basispartikel umfassend ein basisches Basispartikel, das mit einem wasserlöslichen primären Beschichtungsfilm und einem wasserunlöslichen Beschichtungsfilm in dieser Reihenfolge beschichtet ist,
wobei der primäre Beschichtungsfilm von einer Verbindung gebildet oder mindestens eine Verbindung enthält, die aus der Gruppe ausgewählt ist, die aus Zitronensäure, Essigsäure, Weinsäure und einem Salz davon besteht und mindestens eine Verbindung enthält, die aus der Gruppe ausgewählt ist, die aus Hydroxypropylmethylcellulose, Methylcellulose und Hydroxypropylcellulose besteht
und der wasserunlösliche Beschichtungsfilm von einer Verbindung gebildet oder mindestens eine Verbindung enthält, die aus der Gruppe ausgewählt ist, die aus Ethylcellulose, Methacrylsäure-Copolymer, Aminomethacrylsäure-Methacrylat-Copolymer und HydroxypropylmethylcellulosePhthalat besteht.

2. Verfahren zur Herstellung eines Basispartikels umfassend:
Beschichten eines basischen Basispartikels mit einem wasserlöslichen primären Beschichtungsfilm, der mindestens eine Verbindung, die aus der Gruppe ausgewählt ist, die aus Zitronensäure, Essigsäure, Weinsäure und einem Salz davon besteht und mindestens eine Verbindung, die aus der Gruppe ausgewählt ist, die aus Hydroxypropylmethylcellulose, Methylcellulose und Hydroxypropylcellulose besteht, enthält und
Beschichten des erhaltenen Basispartikels mit einem wasserunlöslichen Beschichtungsfilm, der mindestens eine Verbindung enthält, die aus der Gruppe ausgewählt ist, die aus Ethylcellulose, Methacrylsäure-Copolymer, Aminomethacrylsäure-Methacrylat-Copolymer und Hydroxypropylmethylcellulose-Phthalat besteht.

3. Lutschtablette umfassend das Basispartikel gemäß Anspruch 1, wobei die Tablette mit einem pharmazeutisch verträglichen Trägerstoff, einem Lösungsvermittler und/oder einem Gleitmittel tablettiert ist.

## Revendications

1. Particule de base comprenant une particule de base basique revêtue d'un film de revêtement primaire soluble dans l'eau et d'un film de revêtement insoluble dans l'eau, dans cet ordre, dans laquelle le film de revêtement primaire est formé par ou contient au moins un composé sélectionné parmi le groupe composé d'acide citrique, d'acide acétique, d'acide tartrique et d'un sel de ces derniers et au moins un composé sélectionné parmi le groupe composé d'hydroxypropylméthylcellulose, de méthylcellulose et d'hydroxypropylcellulose, et le film de revêtement insoluble dans l'eau est formé par ou contient au moins un composé sélectionné parmi le groupe composé d'éthylcellulose, de copolymère à base d'acide méthacrylique, de copolymère à base de méthacrylate d'acide amino-méthacrylique et de phtalate d'hydroxypropylméthylcellulose.

2. Procédé de fabrication d'une particule de base comprenant:
revêtir une particule de base basique d'un film de revêtement primaire soluble dans l'eau contenant au moins un composé sélectionné parmi le groupe composé d'acide citrique, d'acide acétique, d'acide tartrique et d'un sel de ces derniers et au moins un composé sélectionné parmi le groupe composé d'hydroxypropylméthylcellulose, de méthylcellulose et d'hydroxypropylcellulose, et revêtir la particule de base obtenue d'un film de revêtement insoluble dans l'eau contenant au moins un composé sélectionné parmi le groupe composé d'éthylcellulose de copolymère à base d'acide méthacrylique, de copolymère à base de méthacrylate d'acide amino-méthacrylique et de phtalate d'hydroxypropylméthylcellulose.

3. Comprimé se délitant en bouche comprenant la particule de base selon la revendication 1, dans lequel le comprimé est formé en forme de comprimé avec un excipient acceptable pharmaceutiquement, un agent délitant et/ou un lubrifiant.
